# EUROPEAN PATENT SPECIFICATION

(11) **EP 2 968 696 B1**
(45) Date of publication and mention of the grant of the patent: **26.07.2017**
(21) Application number: 14712077.8
(22) Date of filing: 27.02.2014
(51) Int. Cl.: A61L 31/12, A61L 31/14, A61L 31/16

(54) **MULTI-COMPONENT-FILAMENT-BASED SURGICAL MESHES**
AUF MEHRKOMPONENTENFASERN BASIERENDE CHIRURGISCHE NETZE
MAILLES CHIRURGICALES À BASE DE FILAMENTS À COMPOSANTS MULTIPLES

(30) Priority: 15.03.2013 US 201361788001 P
(43) Date of publication of application: 20.01.2016
(73) Proprietor: Boston Scientific Scimed, Inc., Maple Grove, MN 55311 (US)
(72) Inventor: HARRAH, Timothy, Paul, Cambridge, MA 02139 (US); JIANMIN, Li, Lexington, MA 02421 (US); BODEN, Mark, W., Harrisville, RI 02830 (US)
(74) Representative: Pfenning, Meinig & Partner mbB
(86) International application number: PCT/US2014/019059
(87) International publication number: WO 2014/149500

(56) References cited:
- EP-A2- 2 055 323
- US-A1- 2001 023 020
- US-A1- 2002 052 612
- US-A1- 2006 264 698

## Description

### FIELD OF THE INVENTION

The present invention relates generally to medical articles, and more particularly to meshes for surgical applications.

### BACKGROUND

Pelvic floor disorders are highly prevalent among women. Estimates indicate that around 225,000 women require pelvic organ prolapse (POP) surgery each year in the U.S. and current projections show that one in three women will have to undergo POP surgery by 2050. Pelvic floor disorders involve a dropping down (prolapse) of the bladder, rectum, or uterus caused by weakness of or injury to the ligaments, connective tissue, and muscles of the pelvis. The different types of pelvic floor disorders are named according to the organ affected. For example, a rectocele develops when the rectum drops down and protrudes into the back wall of the vagina. An enterocele develops when the small intestine and the lining of the abdominal cavity (peritoneum) bulge downward between the uterus and the rectum or, if the uterus has been removed, between the bladder and the rectum. A cystocele develops when the bladder drops down and protrudes into the front wall of the vagina. In prolapse of the uterus (procidentia), the uterus drops down into the vagina. Pelvic floor disorders are commonly treated by implanting a surgical mesh within the patient's pelvis to support the organ or organs that require support.

Surgical meshes are also employed in various other soft tissue applications. As one example, urinary incontinence affects millions of men and women of all ages in the United States. Stress urinary incontinence (SUI) affects primarily women and is generally caused by two conditions, intrinsic sphincter deficiency (ISD) and hypermobility. These conditions may occur independently or in combination. In ISD, the urinary sphincter valve, located within the urethra, fails to close properly (coapt), causing urine to leak out of the urethra during stressful activity. Hypermobility is a condition in which the pelvic floor is distended, weakened, or damaged, causing the bladder neck and proximal urethra to rotate and descend in response to increases in intra-abdominal pressure (e.g., due to sneezing, coughing, straining, etc.). The result is that there is an insufficient response time to promote urethral closure and, consequently, urine leakage and/or flow results. A common treatment of SUI is via the use of a surgical mesh, commonly referred to as a sling, which is permanently placed under a patient's bladder neck or mid-urethra to provide a urethral platform. Placement of the sling limits the endopelvic fascia drop, while providing compression to the urethral sphincter to improve coaptation. Further information regarding sling procedures may be found, for example, in the following: Fred E. Govier et al., "Pubovaginal slings: a review of the technical variables," Curr. Opin. Urol. 11:405-410, 2001, John Klutke and Carl Klutke, "The promise of tension-free vaginal tape for female SUI," Contemporary Urol. pp. 59-73, Oct. 2000; and PCT Patent Publication No. WO 00/74633 A2: "Method and Apparatus for Adjusting Flexible Areal Polymer Implants."

US 2001/023020 A1 describes a medical article and an implantable fiber for medical implants. The fiber includes a first component formed from a substantially resorbable material and a second component formed from a fiber-forming polymer.

US 2006/264698 A1 describes a mesh implant which may be utilized for treating urinary incontinence, hernias, uterovaginal prolapses and other related injuries.

EP 2055323 A2 describes filament-reinforced composite fibers which are made from a plurality of filaments and a matrix. These fibers may be used in forming medical devices such as sutures and meshes.

The present invention pertains to meshes that are useful in treating these and other disorders, diseases and conditions.

### SUMMARY OF THE INVENTION

In accordance with the present disclosure, surgical meshes are provided which comprise one or more multi-component polymeric filaments according to claim 1. The multi-component polymeric filaments comprise a filamentous component in the form of multiple sub-filaments and a matrix component in the form of a matrix that binds the sub-filaments into a single filament

There are also described methods of using such surgical meshes.

These and other aspects, embodiments and advantages of the present invention will become immediately apparent to those of ordinary skill in the art upon review of the Detailed Description and claims to follow.

### BRIEF DESCRIPTION OF THE DRAWINGS

Fig. 1 is a schematic illustration of a mesh construction in accordance with an embodiment of the present invention.
Fig. 2 is a schematic illustration of a mesh construction in accordance with another embodiment of the present invention.
Fig. 3 is a schematic illustration of a mesh construction in accordance with yet another embodiment of the present invention.
Fig. 4 is a schematic illustration of a mesh construction in accordance with still another embodiment of the present invention.
Figs. 5A-5F and 6 are schematic cross-sections of several multi-component filament configurations for use in forming the meshes of the present invention.
Fig. 7 illustrates schematic cross-sections of the filaments of Fig. 5A or Fig. 6 upon removal of the matrix component of the multi-component filament.
Figs. 8A and 8B are schematic cross-sections of two so-called "islands in the sea" bi-component filament configurations for use in forming the meshes of the present invention.
Fig. 9 is a schematic illustration of a known system for producing bi-component filaments.
Fig. 10 is a schematic cross-sectional view of a known spin pack for generating a bi-component islands-in-the-sea filament.

### DETAILED DESCRIPTION OF THE INVENTION

A more complete understanding of the present invention is available by reference to the following detailed description of numerous aspects and embodiments of the invention. The detailed description of the invention which follows is intended to illustrate but not limit the invention.

In accordance with the present disclosure, surgical meshes are provided which comprise one or more multi-component polymeric filaments. The multi-component polymeric filaments comprise a filamentous component in the form of multiple sub-filaments and a matrix component in the form of a matrix that binds the sub-filaments into a single filament, as described in the claims.

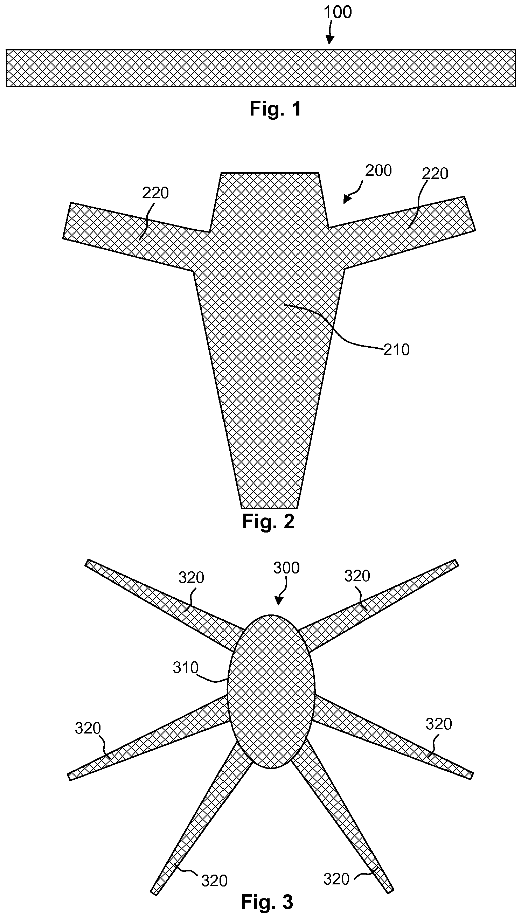

The meshes described herein are typically "substantially two-dimensional" in shape. As used herein a "substantially two-dimensional" object is a sheet-like object, typically one whose length and width are at least 10 times greater than the thickness of the material forming the object, for example, whose length and width are each 10 to 25 to 50 to 100 or more times the thickness. For example, surgical meshes may be in the form of ribbons, sheets, and other more complex sheet-like shapes (see, e.g., Figs. 1 to 4 herein). In certain embodiments, the mesh will be able to take on a planar configuration, for example, when placed on a planar surface such as a table top. However, substantially two-dimensional objects, including the surgical meshes of the present disclosure, need not be planar. For example, such objects may curve in space (e.g., as a substantially two-dimensional orange peel curves around the inner portion of the orange, etc.).

Surgical meshes in accordance with the present disclosure include a wide variety of meshes for soft tissue repair, including meshes for pelvic floor repair, vaginal slings, meshes for renal pelvis repair, urethral slings, hernia meshes (e.g., meshes for inguinal hernia, hiatus hernia, etc.), meshes for thoracic wall defects, breast support meshes and various other soft-tissue surgical support meshes, including meshes for cosmetic and reconstructive surgery, among others. Surgical meshes may be surgically implanted in a variety of subjects, typically vertebrate subjects, more typically mammalian subjects, including human subjects, pets and livestock.

For example, there is schematically illustrated in Fig. 1, a mesh 100, which may be useful as a urethral sling for the treatment of SUI. The material for the mesh 100 comprises one or more multi-component filaments as described herein.

As noted above, pelvic floor (pelvic support) disorders involve a dropping down (prolapse) of the bladder, rectum and/or uterus caused by weakness of or injury to the ligaments, connective tissue, and muscles of the pelvis. As with SUI, treatment of pelvic floor disorders may be treated by implanting a surgical mesh within the patient's pelvis to support the organ or organs that require support.

In accordance with one embodiment, there is schematically illustrated in Fig. 2 a mesh 200, for example, a pelvic floor repair mesh, having a central portion 210 and a plurality of arms 220 that emanate from the central portion 210. As used herein an "arm" is an elongated mesh component whose length is at least two times greater than its width, typically ranging from 2 to 4 to 6 to 8 to 10 or more times the width. (Thus, surgical sling 100 of Fig. 1 can be thought of as a single-arm device.) The material for the mesh 200 comprises one or more multi-component filaments as described herein.

Although the mesh of Fig. 2 has two rectangular arms and a polygonal central body portion, other body and arm shapes and other numbers of arms may be used (e.g., 3, 4, 5, 6, 7, 8, etc.). As one specific variation, Fig. 3 illustrates a mesh 300 having a non-circular (oval) central body portion 310 and six non-rectangular (trapezoidal) arms 320, among near-limitless other possibilities. The material for the mesh 300 comprises one or more multi-component filaments as described herein.

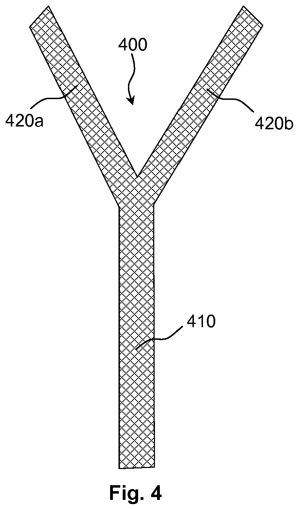

In accordance with another embodiment, there is schematically illustrated in Fig. 4 a surgical mesh 400, for example, a Y-shaped mesh, having arms 410, 420a and 420b. (Arms 420a, 420b may be secured, for example, to the vaginal apex and arm 410 may be secured, for example, to the sacral promontory using sacrocolpopexy procedures known in the art for Y-shaped meshes. The mesh may be placed, for example, using known procedures including transvaginal or abdominal procedures, among others.) The material for the mesh 400 comprises one or more multi-component filaments as described herein.

Meshes in accordance with the present disclosure thus vary widely in shape are useful in performing a variety of procedures, including treatment of stress urinary incontinence (e.g., via a sling procedure, etc.), and pelvic organ prolapse repair, among many others. Depending on the procedure, meshes in accordance with the present disclosure may be implanted by one or more of the following routes, among others: transvaginally (e.g., by vaginal incision), laparoscopically, via open abdominal surgery (laparotomy), transrectally, transcystoscopically, and percutaneously.

Surgical meshes in accordance with the present disclosure are formed using one or more multi-component filaments. Surgical meshes in accordance with the present disclosure include monofilament and multifilament meshes. Surgical meshes in accordance with the present disclosure include woven meshes and non-woven meshes (including knitted meshes, felt meshes, and spunbound meshes, among others).

Surgical meshes in accordance with the present disclosure may have a wide range of pore sizes. In various embodiments, the surgical meshes of the present disclosure may have pore sizes ranging from 1 µm to 2 µm to 5 µm to 10 µm to 25 µm to 50 µm to 75 µm to 100 µm to 0.5 mm to 1 mm to 5 mm to 10 mm to 50 mm to 100 mm in width. The pore size can be varied, for example, to prevent or promote tissue in-growth.

As used herein, a "multi-component filament" is a filament within whose cross-section can be found at least two distinct components, each of different composition, for instance, because the polymer content varies between the components.

Multi-component polymeric filaments for use in the present disclosure are formed from a combination of biostable components and bioabsorbable components. As defined herein, a "biostable" component is one which remains intact over the period that the medical device is intended to remain implanted within the body. Similarly, as defined herein, a "bioabsorbable" component is one that is absorbed by the body, for example, due to dissolution, chemical breakdown, etc. of the component.

Variations in polymer content between the components may be accompanied by variation in a number of physical and chemical characteristics including the following, among others: mechanical strength, hardness, surface tack, elasticity, water diffusivity, therapeutic agent diffusivity (where present), absorption rate (where bioabsorbable), and hydrophobic/hydrophilic nature (influencing, for example, wettability, as well as water diffusivity and therapeutic agent diffusivity, where present).

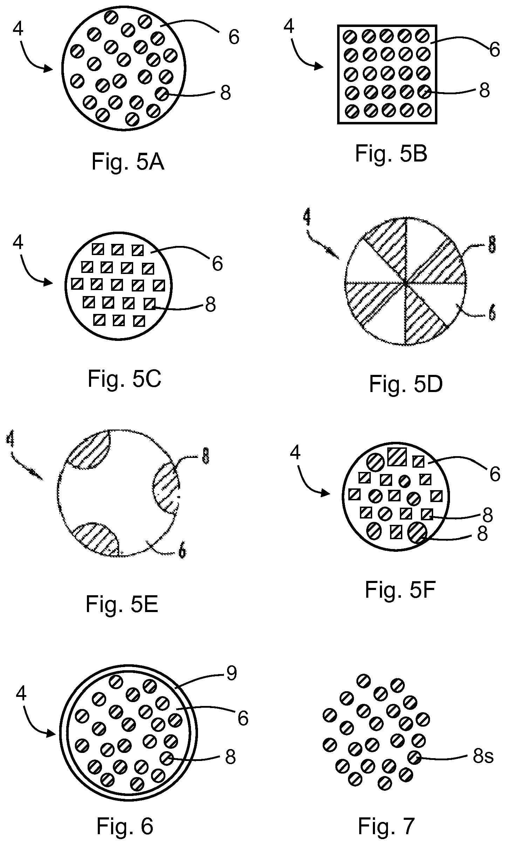

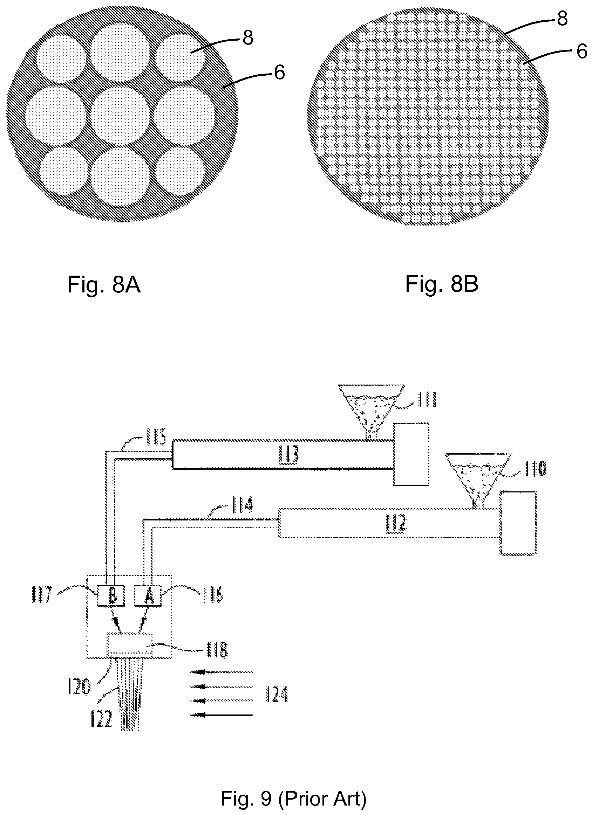

Figs. 5A-5F schematically illustrate several examples of multi-component polymeric filaments 4 for use in the present disclosure. For example, Fig. 5A is an illustration of a multi-component polymeric filament 4 which include a filamentous component 8 in the form of multiple sub-filaments bound together by a matrix component 6. Such a multi-component polymeric filament is sometimes referred to as an "islands-in-the-sea" filament. The number of "islands" (i.e., sub-filaments) in such filaments can range widely as shown in Figs. 8A and 8B. Additional configurations include a filamentous component 8 in the form of multiple sub-filaments bound together by a matrix component 6 are shown in Figs. 5B-5F.

As can be seen from Figs. 5A-5F, the of the sub-filaments can vary in cross-sectional geometry including curvilinear shapes (e.g., circular, oval, eye-shaped, etc.) such as those shown in Figs. 5A, 5B and 5F, polygonal shapes (e.g., triangular, square, trapezoidal, rhomboidal, pentagonal, hexagonal, etc.) such as that shown in Fig. 5C and hybrid linear/curvilinear shapes (e.g., polygons with rounded sides, rounded corners, etc.) such as that shown in Fig. 5D.

It is also noted that sub-filaments having different cross-sectional shapes and different cross-sectional sizes can be provided within a single filament. In this regard, Fig. 5F illustrates a filament with sub-filaments having different cross-sectional shapes (polygonal and curvilinear) and having different cross-sectional areas. Differing sub-filaments within a given filament may also have differing material properties. For example, in a combination of biostable and bioabsorbable sub-filaments the bioabsorbable sub-filaments may have differing bioabsorption rates.

Typically the sub-filaments will extend longitudinally through the matrix component in a fashion that is parallel to the out walls of the filament (i.e., the distance between a given sub-filament and the outer wall of the filament remains constant as the sub-filament proceeds longitudinally through the matrix component). In certain embodiments, however, the sub-filaments will extend longitudinally through the matrix component in a fashion that is non-parallel with the outer walls of the filament (i.e., the distance between a given sub-filament and the outer wall of the filament varies as one proceeds longitudinally through the matrix component). For example, the sub-filaments may run through the matrix component in a wavy or zigzag fashion, among other possibilities. Moreover, the cross-sectional area of a given sub-filament may vary as one proceeds longitudinally along the length of the sub-filament.

The outer cross-sectional geometry of the matrix component can be varied to create a number of overall filament cross-sectional geometries including curvilinear shapes (e.g., circular, oval, eye-shaped, etc.) such as those shown in Figs. 5A and 5C-5F, polygonal shapes (e.g., triangular, square, trapezoidal, rhomboidal, pentagonal, hexagonal, etc.) such as that shown in Fig. 5B, and hybrid linear/curvilinear shapes (e.g., polygons with rounded corners or rounded sides, etc.). In addition, the overall cross-sectional area of a given multi-component filament may vary along the length of the filament.

In certain embodiments, the multi-component filament 4 can include a coating component 9 as shown in Fig. 6

The matrix component (as well as a coating component, if any) is/are bioabsorbable and the filamentous component is biostable. Bioabsorption of the bioabsorbable component(s) result(s) in the formation of multiple sub-filaments. As one example, bioabsorption of the matrix component 6 of the multi-component polymeric filament 4 of Fig. 5A will produce multiple sub-filaments 8s as shown in Fig. 7, wherein the multiple sub-filaments 8s of Fig. 7 correspond to the filamentous component 8 of the multi-component polymeric filament 4 of Fig. 5A. As another example, bioabsorption of the matrix component 6 and coating component 9 of the multi-component polymeric filament 4 of Fig. 6 will also produce multiple sub-filaments 8s as shown in Fig. 7, wherein the multiple sub-filaments 8s of Fig. 7 correspond to the filamentous component 8 of the multi-component polymeric filament 4 of Fig. 6.

Also described is a configuration where the matrix component (as well as a coating component, if any) and the filamentous component are bioabsorbable. The matrix component (as well as a coating component, if any) may be formed of a material or materials that bioabsorb(s) more rapidly than the filamentous component. As one example, initial bioabsorption of the matrix component 6 and coating component 9 of the multi-component polymeric filament 4 of Fig. 6 will initially produce multiple sub-filaments 8s analogous to that shown in Fig. 7, wherein the multiple sub-filaments 8s of Fig. 7 correspond to the filamentous component 8 of the multi-component polymeric filament 4 of Fig. 6. With the passage of additional time, however, the multiple sub-filaments 8s will also eventually bioabsorb. Alternatively, the filamentous component is bioabsorbable and the matrix component is biostable, resulting in channels extending through the matrix material upon bioabsorption of the filamentous component.

In certain embodiments, multi-component filaments are engineered such that the bioabsorbable matrix component (as well as a coating component, if any) is/are bioabsorbed, and the sub-filaments thus released, within a time period ranging from 2 hours to 4 hours to 12 hours to 1 day to 2 days to 4 days to 1 week to 2 weeks to 1 month to 2 months to six months to one year.

The overall width (e.g., diameter of a circular filament, etc.) of the multi-component polymeric filaments of the present disclosure can vary widely, for example, ranging from 10 µm or less to 200 µm or more (e.g., 10 µm to 25 µm to 50 µm to 75 µm to 100 µm to 125 µm to 150 µm to 175 µm to 200 µm), more typically ranging from 10 µm to 200 µm.

The filament width of the sub-filaments of the present disclosure can also vary widely, for example, ranging from 50 nm or less to 50 µm or more (e.g., 50 nm to 100 nm to 250 nm to 500 nm to 1 µm to 2.5 µm to 5 µm to 10 µm to 25 µm to 50 µm).

The number of sub-filaments within the multi-component polymeric filaments of the present disclosure may range, for example from 2 to 1000 or more sub-filaments (2 to 3 to 5 to 10 to 15 to 25 to 50 to 75 to 100 to 150 to 200 to 250 to 500 to 1000 sub-filaments).

By creating meshes with multi-component filaments having a bioabsorbable matrix component and a biostable filament component, meshes can be formed that are able to become softer and more flexible over time following implantation. In this regard, a mesh can be formed which, at the time of implantation, has the look and feel is of conventional mesh. Upon implantation, however, the bioabsorbable matrix component undergoes bioabsorption over a specified period of time, leaving behind a supple multi-filament matrix.

The material for the matrix component, the material for the filamentous component, the overall filament width, the sub-filament width and the number of sub-filaments and will interdependently modulate the final hand-feel of the mesh, the duration of the bioabsorption of the matrix material and the softness of the mesh that remains after bioabsorption.

Classification of meshes include Type I meshes, which are macroporous prostheses (i.e., with pores >75µm), Type II meshes, which are microporous prostheses (i.e., pores <10µ) and Type III meshes, which are macroporous prostheses with multifilamentous or microporous components. Type I meshes are reported to have various advantages over other forms of prosthesis, including Type II or Type III meshes, including enhanced infection resistance, rapid filamentous fixation and host tissue organization. In this regard, and without wishing to be bound by theory, macroporous meshes have been reported to have the following advantages: (a) enhanced resistance to infection, for example, because larger pores allow entry of macrophages and neutrophils, whereas pores or interstices <10 µm can prevent entry of macrophages and neutrophils, while allowing microbes, which can be much smaller than macrophages and neutrophils, to hide and proliferate in the mesh, (b) rapid fibrinous fixation, for example, because macroporous meshes have decreased chance of seroma collection and potential dead spaces between the mesh and the host tissue, promoting formation of proper scaffolding for future fibrocytic infiltration into the mesh, and (c) effective host tissue incorporation, because incorporation of fibroblasts, collagen and blood vessels is effective with pore sizes >75 µm.

Meshes in accordance with the present disclosure can be provided which initially correspond to Type I meshes (e.g., macroporous monofilament meshes in which the monofilament material is a multi- component filament as described herein), but which subsequently convert *in vivo* into Type III meshes (e.g., macroporous meshes with multifilamentous components). Without wishing to be bound by theory, it is believed that a mesh of this type is advantageous in that it can be implanted in an initial configuration that exhibits advantages associated with Type I meshes including, for example, resistance to microbial infection and/or enhanced integration into host tissue. Such a mesh may also be provided with an initial configuration having sufficient stiffness to prevent implant migration and folding. At a later time, after in vivo bioabsorption of bioabsorbable component(s) of the mesh (e.g., , bioabsorbable matrix components and any other bioabsorbable mesh components such as bioabsorbable coating components), the sub-filaments are released from the matrix component and the mesh takes on a supple multi-filamentous configuration within the body.

The physical properties of the multi-filamentous mesh material that remains in the body may be matched with the physical properties of the surrounding/adjacent tissue, including one or more of the following physical properties among others: hardness/softness, elasticity, stiffness/flexibility and strength. As an example, it may be desirable to match the hardness of the multi-filamentous mesh material with the hardness of surrounding/adjacent tissue (e.g., the durometer of the sub-filaments may be matched with the durometer of the tissue). As another example, it may be desirable to match the elastic modulus of the multi-filamentous mesh material with the elastic modulus of surrounding/adjacent tissue (e.g., the Young's modulus of the sub-filaments may be matched with the Young's modulus of the tissue). Physical properties may be matched, for example, to provide for more normal load bearing and/or to help stimulate proper cell growth and remodeling (e.g., stimulation of fibroblasts to generate collagen, and proper conversion of collagen III to collagen I), among other possible benefits.

It should be noted that meshes may be formed in accordance with the present disclosure that include filaments in addition to the multi-component filaments described herein.

For example, as noted above, meshes in accordance with the present disclosure include those that comprise a body portion and a plurality of arms extending from the body. Such meshes may be used, for example, for pelvic organ prolapse repair. In certain of these embodiments, all or a portion of the body portion (which may be in contact one or more pelvic organs upon implantation) may be formed from multi-component filaments like those described herein. On the other hand, all or a portion of the arms may be made of a purely biostable material.

Materials for forming meshes in accordance with the present disclosure include various synthetic biostable polymers, various synthetic bioabsorbable polymers, various naturally occurring biostable polymers and various naturally occurring bioabsorbable polymers. A combination of biostable and bioabsorbable polymers is employed in various embodiments.

As used herein, "polymers" are molecules containing multiple copies (e.g., from 2 to 5 to 10 to 25 to 50 to 100 to 250 to 500 to 1000 or more copies) of one or more constitutional units, commonly referred to as monomers. Polymers may take on a number of architectures, which may be selected, for example, from cyclic, linear and branched architectures. Branched architectures include star-shaped architectures (e.g., configurations in which three or more chains emanate from a single branch point), comb architectures (e.g., architectures having a main chain and a plurality of side chains), and dendritic architectures (e.g., arborescent and hyperbranched polymers), among others.

As used herein, "homopolymers" are polymers that contain multiple copies of a single constitutional unit. "Copolymers" are polymers that contain multiple copies of at least two dissimilar constitutional units, examples of which include random, gradient, periodic (e.g., alternating) and block copolymers. As used herein, "block copolymers" are copolymers that contain two or more polymer blocks that differ in composition, for instance, because a constitutional unit (i.e., monomer) is found in one polymer block that is not found in another polymer block. As used herein, a "polymer block" is a grouping of constitutional units (e.g., 2 to 5 to 10 to 25 to 50 to 100 to 250 to 500 to 1000 or more units). Blocks can be branched or unbranched. Blocks can contain a single type of constitutional unit (also referred to herein as "homopolymeric blocks") or they can contain multiple types of constitutional units (also referred to herein as "copolymeric blocks") which may be provided, for example, in a random, gradient, or periodic (e.g., alternating) distribution.

Examples of synthetic biostable polymers may be selected from the following: (a) polyolefin homopolymers and copolymers, including homopolymers and copolymers of C2-C8 alkenes, for example, polyethylene and polypropylene among others, (b) fluoropolymers, including homopolymers and copolymers of C2-C8 alkenes in which one or more hydrogen atoms are substituted with fluorine, for example, polytetrafluoroethylene (PTFE), polyvinylidene fluoride (PVDF), poly(vinylidene fluoride-*co*-hexafluoropropene) (PVDF-HFP) among others, (c) polyamides such as nylons, among others, (d) polyesters, including, for example, polyethylene terephthalate, among others, (e) polyurethanes such as polyisobutylene based polyurethanes (PIB-PU) that comprise one or more polyisobutylene segments, among others, (f) polyoxyalkylenes including homopolymers of trioxane (polytrioxane, also known as polyoxymethylene or acetal) and copolymers of trioxane (e.g., copolymers of trioxane and dioxane), (g) styrenic copolymers such as alkene-styrene copolymers, including block copolymers comprising one or more polystyrene blocks and one or more polyalkene blocks, for instance, poly(styrene-*b*-isobutylene-*b*-styrene) (SIBS), poly(styrene-*b*-ethylene/butylene-*b*-styrene) (SEBS) among others, (h) as well as various other non-absorbable polymers and copolymers (including block copolymers).

Examples of synthetic bioabsorbable polymers may be selected, for example, from polyesters and polyanhydrides, among others. Specific bioabsorbable polymers may be selected from suitable members of the following, among others: (a) polyester homopolymers and copolymers (including polyesters and poly[ester-amides]), such as polyglycolide, polylactide (PLA), including poly-L-lactide, poly-D-lactide, and poly-D,L-lactide, poly(lactide-co-glycolide) (PLG), including poly(L-lactide-co-glycolide), poly(D-lactide-co-glycolide) and poly(D,L-lactide-co-glycolide), poly(beta-hydroxybutyrate), poly-D-gluconate, poly-L-gluconate, poly-D,L-gluconate, poly(epsilon-caprolactone), poly(delta-valerolactone), poly(p-dioxanone), poly(trimethylene carbonate), poly(lactide-co-delta-valerolactone), poly(lactide-co-epsilon-caprolactone), poly(lactide-co-beta-malic acid), poly(lactide-co-trimethylene carbonate), poly(glycolide-co-trimethylene carbonate), poly(beta-hydroxybutyrate-co-beta-hydroxyvalerate), poly[1,3-bis(p-carboxyphenoxy)propane-co-sebacic acid], poly(sebacic acid-co-fumaric acid), and poly(ortho esters) such as those synthesized by copolymerization of various diketene acetals and diols, among others; and (b) polyanhydride homopolymers and copolymers such as poly(adipic anhydride), poly(suberic anhydride), poly(sebacic anhydride), poly(dodecanedioic anhydride), poly(maleic anhydride), poly[1,3-bis(p-carboxyphenoxy)methane anhydride], and poly[alpha,omega-bis(p-carboxyphenoxy)alkane anhydrides] such as poly[1,3-bis(p-carboxyphenoxy)propane anhydride] and poly[1,6-bis(p-carboxyphenoxy)hexane anhydride], among others.

Where a bioabsorbable polyester is used (e.g., PLA, PLG, etc.), one or more soft blocks, for example, polyethylene oxide (PEO), poly(trimethylene carbonate) (PTMC), poly(dioxane-2-one) (PPDO) or polycaprolactone (PCL) blocks, among others, may be included with one or more polyester blocks in the polymer to vary hardness, elongation, and degradation rate of the polymer. Examples include diblock and triblock copolymers such as PLA-PCL, PLA-PCL-PLA, PLG-PCL, PLG-PCL-PLG, PLA-PEO, PLA-PEO-PLA, PLG-PEO, PLG-PEO-PLG, PCL-PLA-PTMC, PLA-PTMC-PCL and PLA-PTMC-PPDO, among others.

Where copolymers are employed, copolymers with a variety of monomer ratios may be available. For example, where isobutylene-styrene copolymers (e.g., SIBS) are used, the ratio of monomers in these polymers can be selected to obtain mechanical properties such that tissue compatibility is enhanced. For instance, a higher isobutylene content will result in a softer polymer.

As another example, where PLG is used, a variety of lactide:glycolide molar ratios will find use herein, and the ratio is largely a matter of choice, depending in part on the rate of degradation desired. For instance, a 50:50 PLG polymer, containing 50% D,L-lactide and 50% glycolide, will provide a faster resorbing copolymer, while 75:25 PLG degrades more slowly, and 85:15 and 90:10, even more slowly, due to the increased lactide component. Degradation rate can also be controlled by such factors as polymer molecular weight and polymer crystallinity. More broadly, where used, PLG copolymers include those having a lactide/glycolide molar ratio ranging, for example, from 10:90 or less to 15:85 to 20:80 to 25:75 to 40:60 to 45:55 to 50:50 to 55:45 to 60:40 to 75:25 to 80:20 to 85:15 to 90:10 or more.

Examples of naturally occurring polymers include biostable and bioabsorbable polymers such as cellulose including biosynthesized cellulose, alginic acid, hyaluronic acid, and collagen, among many others.

Polymers employed herein include non-crosslinked and crosslinked (e.g., ionically crosslinked, covalently crosslinked, etc.) polymers.

In various embodiments, the matrix component, the filamentous component, and the coating component (if any) of the multi-component filament may each independently contain 25 wt% or more of one or more of the preceding polymers (e.g., from 25 wt% to 40 wt% to 50 wt% to 60 wt% to 70 wt% to 80 wt% to 90 wt% to 95 wt% to 98 wt% to 99 wt% or more).

In certain embodiments, the multi-component filaments of the present disclosure comprise various additional agents (i.e., agents in addition to the polymeric materials that are used to form the multi-component filaments) including therapeutic agents, plasticizers and imaging agents, among other possible agents. The matrix component, the filamentous component and the coating component (if any) of the multi-component filament may each independently comprise such additional agents.

"Therapeutic agents," drugs," "bioactive agents" "pharmaceuticals," "pharmaceutically active agents" and other related terms may be used interchangeably herein. Therapeutic agents may be used singly or in combination.

In certain embodiments, the multi-component filaments of the present disclosure comprise one or more therapeutic agents, for example, selected from the following, among many others: (a) female hormones such as estrogen (including estrogen cocktails) and progesterone, (b) anti-inflammatory agents (e.g., for purposes of reducing macrophage levels, resulting in less muscle regeneration and re-growth and less scarring or filamentous capsule formation) including corticosteroids such as hydrocortisone and prednisolone, and non-steroidal anti-inflammatory drugs (NSAIDS) such as aspirin, ibuprofen, and naproxen; (c) narcotic and non-narcotic analgesics and local anesthetic agents (e.g., for purposes of minimizing pain); (d) growth factors such as epidermal growth factor and transforming growth factor-α (e.g., for purposes of stimulate the healing process and or promoting growth of collagenous tissue); (e) antimicrobial agents including chlorhexidine, triclosan, nitrofurazone, benzalkonium chlorides, silver salts, silver particles, metallic silver and antibiotic agents such as the penicillins (e.g., penicillin G, methicillin, oxacillin, ampicillin, amoxicillin, ticarcillin, etc.), the cephalosporins (e.g., cephalothin, cefazolin, cefoxitin, cefotaxime, cefaclor, cefoperazone, cefixime, ceftriaxone, cefuroxime, etc.), the carbapenems (e.g., imipenem, metropenem, etc.), the monobactems (e.g., aztreonem, etc.), the carbacephems (e.g., loracarbef, etc.), the glycopeptides (e.g., vancomycin, teichoplanin, etc.), bacitracin, polymyxins, colistins, fluoroquinolones (e.g., norfloxacin, lomefloxacin, fleroxacin, ciprofloxacin, enoxacin, trovafloxacin, gatifloxacin, etc.), sulfonamides (e.g., sulfamethoxazole, sulfanilamide, etc.), diaminopyrimidines (e.g., trimethoprim, etc.), rifampin, aminoglycosides (e.g., streptomycin, neomycin, netilmicin, tobramycin, gentamicin, amikacin, etc.), tetracyclines (e.g., tetracycline, doxycycline, demeclocycline, minocycline, etc.), spectinomycin, macrolides (e.g., erythromycin, azithromycin, clarithromycin, dirithromycin, troleandomycin, etc.), and oxazolidinones (e.g., linezolid, etc.), (f) pharmaceutically acceptable salts, esters and other derivatives of the foregoing, and (g) combinations of two or more of the foregoing.

In certain specific embodiments, the multi-component filaments of the present disclosure comprise two or more antimicrobial agents, for example, two or more antibiotic agents. Such substances may be provided, for example, to reduce the risk of microbial infection (including biofouling of the mesh and infection in surrounding tissue) upon implantation of the mesh, among other purposes. For example, in some embodiments, meshes can be prepared with an antibiotic cocktail that will be effective against skin-borne pathogens, particularly *Staphylococcus aureus* (*S. aureus*), including Methicillin-resistant *Staphylococcus aureus* (MRSA), and *Escherichia coli* (*E. coli*), reducing the risk of infection (including infection by such organisms) upon implantation of the mesh. One antibiotic combination that has proven effective against a broad spectrum of skin-borne pathogens is minocycline and rifampin. As noted above, the matrix component, the filamentous component and the coating component (if any) of the multi-component filament may each independently comprise such agents. Preferably, the matrix component, the coating component (if any), or both will comprise such agents.

Additional agents for use in conjunction with the multi-component filaments of the present disclosure also include imaging agents such as (a) contrast agents for use in connection with x-ray fluoroscopy, including metals, metal salts and oxides (particularly bismuth salts and oxides), and iodinated compounds, among others, (b) contrast agents for use in conjunction with ultrasound imaging, including organic and inorganic echogenic particles (i.e., particles that result in an increase in the reflected ultrasonic energy) or organic and inorganic echolucent particles (i.e., particles that result in a decrease in the reflected ultrasonic energy), and (c) contrast agents for use in conjunction with magnetic resonance imaging (MRI), including contrast agents that contain elements with relatively large magnetic moment such as Gd(III), Mn(II), Fe(III) and compounds (including chelates) containing the same, such as gadolinium ion chelated with diethylenetriaminepentaacetic acid.

Additional agents for use in conjunction with the multi-component filaments of the present disclosure further include plasticizers, which may be selected from one or more of the following organic plasticizers, among others: citrate esters such as tributyl, triethyl, triacetyl, acetyl triethyl, and acetyl tributyl citrate (ATBC), dioxane, phthalate derivatives such as dimethyl phthalate, diethyl phthalate and dibutyl phthalate, glycerol, glycols such as polypropylene glycol, propylene glycol, polyethylene glycol and ethylene glycol, surfactants such as sodium dodecyl sulfate and polyoxymethylene (20) sorbitan and polyoxyethylene (20) sorbitan monooleate. Such plasticizers may be provided for better handling and a more robust polymeric matrix (e.g., in one or more of the matrix component, the filamentous component, and the coating component, if any, of the multi-component filament).

In various embodiments, the matrix component, the filamentous component and the coating component (if any) of the multi-component filament may each independently contain 1 wt% or more of one or more of the preceding additional agents (e.g., from 1 wt% to 2 wt% to 5 wt% to 10 wt% to 25 wt% to 40 wt% to 50 wt% to 60 wt% to 75 wt% or more).

Multi-component filaments for the practice of the present disclosure, including multi-component polymeric filaments, may be formed by any suitable filament forming technique, including, for example, melt spinning and solvent spinning (e.g., dry spinning and wet spinning). These processes typically employ extrusion nozzles having one or more orifices, also called distributors, jets or spinnerets. Filaments having a variety of cross-sectional configurations (see, e.g., Figs. 5A-5F, among many other possibilities) may be formed, depending upon the shape of the orifice(s). In melt spinning, polymers are heated to melt temperature prior to extrusion. In wet and dry spinning polymers are dissolved in a solvent prior to extrusion. In dry spinning, the extrudate is subjected to conditions whereby the solvent is evaporated, for example, by exposure to a vacuum or heated atmosphere (e.g., air) which removes the solvent by evaporation. In wet spinning the spinneret is immersed in a liquid, and as the extrudate emerges into the liquid, it solidifies. The resulting filament is typically taken up on a rotating mandrel or another take-up device. During take up, the filament may be stretched (i.e., drawn) to orient the polymer molecules. A common aspect to various spinning techniques, including those described above, is that a polymer-containing liquid is extruded and ultimately solidified (e.g., due to cooling, solvent removal, chemical reaction, etc.).

An example of a system 100 for thermally (melt) producing multi-component filaments (specifically, bi-component filaments) may be found, for example, in Fig. 9. Turning now to Fig. 9, the system 100 includes a first hopper 110 into which pellets of a polymer component A are placed. The polymer is fed from hopper 110 to screw extruder 112, where the polymer is melted. The molten polymer flows through heated pipe 114 into metering pump 116 and spin pack 118. A second hopper 111 feeds a polymer component B into a screw extruder 113, which melts the polymer. The molten polymer flows through heated pipe 115 and into a metering pump 117 and spin pack 118. Spin pack 118 includes a spinneret 120 with orifices through which bi-component filaments 122 are extruded. One example of a suitable spin pack that may be utilized for the formation of bi-component filaments is described in U.S. Pat. No. 5,162,074 to Hills. The extruded filaments 122 emerging from the spinneret may be quenched with a quenching medium 124 (e.g., air) and may be directed, for example, onto a spinning mandrel (not shown) or any other suitable take-up arrangement.

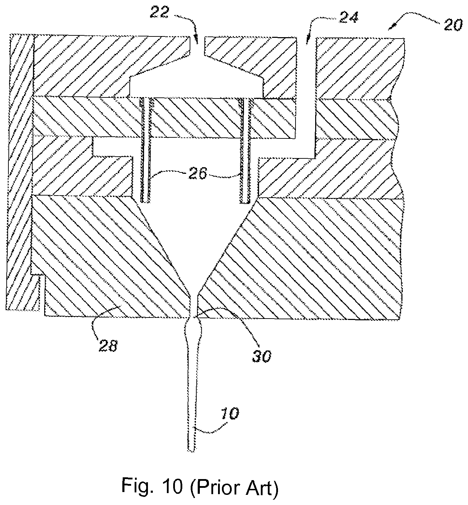

A cross-sectional view of a spin pack 20 which is suitable for generating a bi-component islands-in-the-sea filament 10 is illustrated in Fig. 10. Spin pack 20 includes upstream openings 22 and 24 through which the "island" polymer and "sea" polymer, respectively, enter spin pack 20. The island polymer travels through capillary tubes 26 which inject the island polymer into the flow of the sea polymer at the entrance to spinneret 28 in various discrete locations (only two locations are shown in the cross-section, but more are present along the edge of the spinneret entrance). The polymers flow through the spinneret 28 to an orifice 30, where they are extruded as an islands-in-the-sea filament 10.

While only two polymer component streams are depicted in Figs. 9 and 10, it is noted that systems may be configured for processing any selected number of polymer streams (e.g., three, four or more) depending upon the desired filament configuration. Moreover, although melt spinning is specifically used to form the filaments in these drawings, other types of filament spinning processes may be employed, including dry spinning, wet spinning, and chemical-reaction-based spinning, among others.

Further information concerning multi-component filament spinning processes can be found, for example, in U.S. Patent Nos. 4,381,274 to Kessler et al., 4,370,114 to Okamoto et al., 5,162,074 to Hills, 5,344,297 to Hills, 6,551,353 to Baker et al., 6,767,498 to Talley Jr. et al., 6,803,102 to Talley et al., 6,833,104 to Berger, 6,861,142 to Wilkie et al.

Although multi-component filament spinning processes may be preferred in many instances, in other embodiments, the matrix may be added to a previously formed group of sub-filaments.

As noted above, surgical meshes that may be formed using multi-component filaments in accordance with the present disclosure are varied and include two-dimensional (i.e., open volume) structures and three-dimensional (i.e., closed volume) structures, among others. They may be formed from multi-component filaments using any suitable filament-based construction technique including, for example, various woven and non-woven (e.g., knitted, braided, coiled, randomly wrapped, etc.) techniques. Examples of non-woven techniques include those that utilize thermal fusion, fusion due to removal of residual solvent, mechanical entanglement, chemical binding, adhesive binding, and so forth.

Although various embodiments are specifically illustrated and described herein, it will be appreciated that modifications and variations of the present disclosure are covered by the above teachings and are within the purview of the appended claims.

## Claims

1. A surgical mesh comprising a multi-component polymeric filament which comprises a filamentous component in the form of multiple sub-filaments including biostable sub-filaments and bioabsorbable sub-filaments and a matrix component that joins the sub-filaments into a single filament, wherein the filamentous component comprises a biostable polymer and the matrix component comprises a bioabsorbable polymer such that the multi-component polymeric filament forms a plurality of distinct small filaments corresponding to said biostable sub-filaments upon absorption of the bioabsorbable polymer of the matrix component and absorption of the bioabsorbable sub-filaments in vivo.

2. The surgical mesh of claim 1, wherein the biostable polymer is selected from polyolefin homopolymers and copolymers, fluoropolymer homopolymers and copolymers, and polyurethanes.

3. The surgical mesh of claim 1, wherein the biostable polymer is polypropylene.

4. The surgical mesh of any of claims 1-3, wherein the bioabsorbable polymer is selected from polyester homopolymers and copolymers or wherein the bioabsorbable polymer is poly(lactide-co-glycolide).

5. The surgical mesh of any of claims 1-4, wherein the matrix component, the filamentous component, or both, further comprises a plasticizer.

6. The surgical mesh of any of claims 1-5, wherein the multi-component polymeric filament further comprises a bioabsorbable coating layer over the matrix component.

7. The surgical mesh of any of claims 1-6, wherein the multi-component polymeric filament ranges from 10 µm to 200 µm in width.

8. The surgical mesh of any of claims 1-7, wherein the sub-filaments range from 1 µm to 50 µm in width.

9. The surgical mesh of any of claims 1-8, the multi-component polymeric filament comprises from 2 to 500sub-filaments.

10. The surgical mesh of any of claims 1-9, wherein the mesh has a pore width of 75 µm or more.

11. The surgical mesh of any of claims 1-10, wherein the matrix component further comprises a therapeutic agent.

12. The surgical mesh of any of claims 1-11, wherein the mesh comprises a body and two or more arms extending from the body or wherein the mesh comprises three or more arms.

13. The surgical mesh of any of claims 1-12, wherein the mesh is a woven mesh or wherein the mesh is a non-woven mesh.

14. The surgical mesh of any of claims 1-13, wherein the bioabsorbable sub-filaments have differing absorption rates.

## Patentansprüche

1. Chirurgisches Netz, aufweisend eine Mehrkomponenten-Polymerfaser, die eine faserige Komponente in der Form mehrerer Subfasern, einschließlich biostabiler Subfasern und bioabsorbierbarer Subfasern, und eine Matrixkomponente, die die Subfasern in eine einzelne Faser vereinigt, enthält, wobei die faserige Komponente ein biostabiles Polymer aufweist und die Matrixkomponente ein bioabsorbierbares Polymer derart aufweist, dass die Mehrkomponenten-Polymerfaser mehrere getrennte kleine Fasern entsprechend den biostabilen Subfasern bei Absorption des bioabsorbierbaren Polymers der Matrixkomponente und Absorption der bioabsorbierbaren Subfasern in vivo bildet.

2. Chirurgisches Netz nach Anspruch 1, bei dem das biostabile Polymer ausgewählt ist aus Polyolefin-Homopolymeren und -Copolymeren, Fluorpolymer-Homopolymeren und -Copolymeren und Polyurethanen.

3. Chirurgisches Netz nach Anspruch 1, bei dem das biostabile Polymer Polypropylen ist.

4. Chirurgisches Netz nach einem der Ansprüche 1 - 3, bei dem das bioabsorbierbare Polymer ausgewählt ist aus Polyester-Homopolymeren und -Copolymeren oder bei dem das bioabsorbierbare Polymer Poly(lactid-co-glycolid) ist.

5. Chirurgisches Netz nach einem der Ansprüche 1 - 4, bei dem die Matrixkomponente, die faserige Komponente oder beide weiterhin einen Weichmacher aufweisen.

6. Chirurgisches Netz nach einem der Ansprüche 1 - 5, bei dem die Mehrkomponenten-Polymerfaser weiterhin eine bioabsorbierbare Überzugsschicht über der Matrixkomponente aufweist.

7. Chirurgisches Netz nach einem der Ansprüche 1 - 6, bei dem die Mehrkomponenten-Polymerfaser eine Breite im Bereich von 10 µm bis 200 µm hat.

8. Chirurgisches Netz nach einem der Ansprüche 1 - 7, bei dem die Subfasern eine Breite im Bereich von 1 µm bis 50 µm haben.

9. Chirurgisches Netz nach einem der Ansprüche 1 - 8, bei dem die Mehrkomponenten-Polymerfaser von 2 bis 500 Subfasern aufweist.

10. Chirurgisches Netz nach einem der Ansprüche 1 - 9, bei dem das Netz eine Porenbreite von 75 µm oder mehr hat.

11. Chirurgisches Netz nach einem der Ansprüche 1 - 10, bei dem die Matrixkomponente weiterhin ein therapeutisches Mittel aufweist.

12. Chirurgisches Netz nach einem der Ansprüche 1 - 11, bei dem das Netz einen Körper und zwei oder mehr Arme, die sich von dem Körper weg erstrecken, aufweist oder bei dem das Netz drei oder mehr Arme aufweist.

13. Chirurgisches Netz nach einem der Ansprüche 1 - 12, bei dem das Netz ein gewebtes Netz ist oder bei dem das Netz ein nichtgewebtes Netz ist.

14. Chirurgisches Netz nach einem der Ansprüche 1 - 13, bei dem die bioabsorbierbaren Subfasern unterschiedliche Absorptionsraten haben.

## Revendications

1. Maillage chirurgical qui comprend un filament polymérique à multiples composants qui comprend un composant filamenteux sous la forme de multiples sous-filaments qui incluent des sous-filaments bio-stables et des sous-filaments bio-absorbables et un composant sous forme de matrice qui joint les sous-filaments selon un unique filament, dans lequel le composant filamenteux comprend un polymère bio-stable et le composant sous forme de matrice comprend un polymère bio-absorbable de telle sorte que le filament polymérique à multiples composants forme une pluralité de petits filaments distincts qui correspondent auxdits sous-filaments bio-stables suite à l'absorption du polymère bio-absorbable du composant sous forme de matrice et à l'absorption des sous-filaments bio-absorbables in vivo.

2. Maillage chirurgical selon la revendication 1, dans lequel le polymère bio-stable est sélectionné parmi les homopolymères et copolymères de polyoléfine, les homopolymères et copolymères de fluoropolymère, et les polyuréthanes.

3. Maillage chirurgical selon la revendication 1, dans lequel le polymère bio-stable est du polypropylène.

4. Maillage chirurgical selon l'une quelconque des revendications 1 à 3, dans lequel le polymère bio-absorbable est sélectionné parmi les homopolymères et copolymères de polyester ou dans lequel le polymère bio-absorbable est du poly(lactide-co-glycolide).

5. Maillage chirurgical selon l'une quelconque des revendications 1 à 4, dans lequel le composant sous forme de matrice, le composant filamenteux ou les deux comprend/comprennent en outre un plastifiant ou agent de plastification.

6. Maillage chirurgical selon l'une quelconque des revendications 1 à 5, dans lequel le filament polymérique à multiples composants comprend en outre une couche de revêtement bio-absorbable au-dessus du composant sous forme de matrice.

7. Maillage chirurgical selon l'une quelconque des revendications 1 à 6, dans lequel le filament polymérique à multiples composants présente une largeur qui s'inscrit dans la plage de 10 µm à 200 µm.

8. Maillage chirurgical selon l'une quelconque des revendications 1 à 7, dans lequel les sous-filaments présentent une largeur qui s'inscrit dans la plage de 1 µm à 50 µm.

9. Maillage chirurgical selon l'une quelconque des revendications 1 à 8, dans lequel le filament polymérique à multiples composants comprend de 2 à 500 sous-filaments.

10. Maillage chirurgical selon l'une quelconque des revendications 1 à 9, dans lequel le maillage présente une largeur de pore de 75 µm ou plus.

11. Maillage chirurgical selon l'une quelconque des revendications 1 à 10, dans lequel le composant sous forme de matrice comprend en outre un agent thérapeutique.

12. Maillage chirurgical selon l'une quelconque des revendications 1 à 11, dans lequel le maillage comprend un corps et deux bras ou plus qui s'étendent depuis le corps ou dans lequel le maillage comprend trois bras ou plus.

13. Maillage chirurgical selon l'une quelconque des revendications 1 à 12, dans lequel le maillage est un maillage tissé ou dans lequel le maillage est un maillage non tissé.

14. Maillage chirurgical selon l'une quelconque des revendications 1 à 13, dans lequel les sous-filaments bio-absorbables présentent des taux/vitesses d'absorption différent(e)s.
